# EUROPEAN PATENT SPECIFICATION

(11) **EP 3 231 407 B1**
(45) Date of publication and mention of the grant of the patent: **10.03.2021**
(21) Application number: 16305441.4
(22) Date of filing: 15.04.2016
(51) Int. Cl.: A61G 7/057, A61B 5/11

(54) **SUPPORT DEVICE WITH SENSING ELEMENTS**
TRÄGERVORRICHTUNG MIT SENSORELEMENTEN
DISPOSITIF DE SUPPORT COMPORTANT DES ÉLÉMENTS DE DÉTECTION

(43) Date of publication of application: 18.10.2017
(73) Proprietor: Hill-Rom Services, Inc., Batesville, IN 47006 (US)
(72) Inventor: Flocard, Thierry, 56330 Pluvigner (FR); Kaikenger, Philippe, 56330 Pluvigner (FR); Duvert, Jean-Bernard, 56330 Pluvigner (FR)
(74) Representative: Vleck, Jan Montagu

(56) References cited:
- EP-A1- 2 031 362
- EP-A2- 2 702 972
- US-A1- 2009 000 614
- US-A1- 2015 128 354
- US-B2- 8 598 893

## Description

The present invention relates to a support device comprising at least one sensing element. In particular, the present invention relates to a support device comprising a sensor pad comprising a plurality of sensing elements. Particular embodiments of the invention may be used in hospital or long-care beds.

Patients lying on support devices, such as hospital bed mattresses, for extended periods of time are susceptible to the development of pressure ulcers (also known as decubitus ulcers or bedsores). Pressure ulcers oftentimes are lesions found adjacent to bony or cartilaginous areas. While many devices and methods have been developed to reduce the occurrence of pressure ulcers, there is still room for improvement. Thus there remains a need for further contributions in this area.

One of the proven techniques for preventing the formation and development of bedsores in patients consists of supporting the patients on beds comprising inflatable air-filled cell mattresses in which the air pressure is regulated relative to the morphology and weight of the patient with the aim of minimizing the interface pressures between the patient's body and the surface of the mattress. From load data exerted by the patient on a given mattress in a particular area such as the sacral region, the ideal indentation profile of the patient over the entire surface of the mattress can be deduced and hence the value of the internal air pressure in the mattress can be adjusted for obtaining an ideal support of the patient on the mattress.

US 2009/0056020 A1, in the name of Caminade, describes a pressure sensor and a support device including such a pressure sensor, for detecting and measuring at least one bearing pressure applied to a support device, in which said pressure sensor comprises at least one resistive force-detector cell placed between at least two protective plates suitable for avoiding any pressure bearing at a point or along a line directly against a surface of the resistive cell and for converting such a pressure into uniform pressure over the entire area of the resistive cell.

US 2015/0128354 A1, in the name of Enhanced Surface Dynamics, Inc., describes a resting body support, such as a mattress or an encasement for a resting body support, comprising a sensor layer which may incorporate a mattress adjustment system. The system may comprise at least one pressure detection mat comprising a plurality of sensors arranged in a matrix and at least one processor configured to monitor a plurality of pressure values detected by each of said sensors; and at least one active mattress comprising a plurality of cells, the cells being controlled by an inflation control unit that is connected to said processor, and operable to individually adjust the level of inflation of said plurality of cells based on said plurality of pressure values.

It is also known in medical fields that sensor systems included within a person's bed can provide non-invasive monitoring of the person over long time-periods. However, there do not exist such devices for all aspects of a person's status that are accurate, convenient for use and cheap to produce. Many of the current person monitoring systems also require regular checking by nurses or care-givers. This can cause problems due to a lack of continuous observation, especially during the night. Therefore, in-bed sensor systems are not currently used commonly, and the medical profession is still dependent on external, and often bulky and invasive devices to monitor persons.

It would be desirable to provide a convenient, inexpensive and non-invasive in-bed sensor system to detect load exerted by a patient on a mattress and to monitor multiple aspects of a patient's status. It would also be desirable to reduce the number of in-bed sensing elements required to monitor a patient's status.

According to a first aspect of the present invention, there is provided a support device for supporting the body of a person, the support device comprising: an inflatable layer formed of a plurality of inflatable cells communicating with inflation and deflation means; a sensor pad arranged below the inflatable layer, the sensor pad comprising at least three sensing elements configured to sense the load applied to the or each sensing element; each sensing element being a capacitive sensing element; and electric circuitry connected to the or each sensing element of the sensor pad. The three sensing elements are separated from one another in a direction along the width of the support device, and the three sensing elements comprise: a left sensing element arranged below a left hand portion of the inflatable layer, a middle sensing element arranged below a central portion of the inflatable layer, and a right sensing element arranged below a right hand portion of the inflatable layer The electric circuitry is configured to: control the inflation and deflation means for filling or emptying, respectively, the inflatable cells of the inflatable layer in such a way that the internal air pressure of the inflatable cells is a function of the load of the body of a person resting on the support device as sensed by the or each sensing element, and determine the position on the support device of a person resting on the support device based on the load sensed by the or each sensing element. The electric circuitry is further configured to combine the load sensed by each sensing element and determine the total load of a person resting on the support device based on the combined load measurements; compare the load sensed by the middle sensing element to the determined total load of a person resting on the support device; and determine that the person resting on the support device is in an exit position on the support device if the load sensed by the middle sensing element is less than a predetermined fraction of the determined total load.

The sensing elements in the sensor pad sense the load exerted by a person resting on the support device and the electric circuitry is configured to use measurements of the load from the sensor pad to both control the inflation and deflation of the cells of the inflatable layer and to determine the position on the support device of the person resting on the support device. This reduces the number of sensor systems required to monitor the status of a person resting on the support device. This may reduce the impact of the sensor systems on a person resting on the support device. This may improve the comfort of a person resting on the support device. This may also reduce the cost of the sensor systems.

The load of the body of a person resting on the support device may be spread over a portion of an upper surface of the support device. The upper surface of the support device may be an upper surface of the inflatable layer. The inflatable layer of the support device is arranged between the body of a person resting on the support device and the sensor pad. As such, the load experienced by the sensor pad may comprise at least the load of the body of the person resting on the support device and the load of the inflatable layer. The load applied to the sensing elements may also depend on the inflation of the inflatable layer.

The load applied to the sensing elements is the load from a lower surface of the portion of the support device arranged directly above the sensing elements. This may be referred to as the 'sensed surface'. The lower surface of the support device arranged directly above the sensing element, or the sensed surface, may be a lower surface of the inflatable layer. The portion of the support device arranged above the sensing elements may spread the load of the body of a person resting on the upper surface of the support device between the upper surface and the lower surface, such that the load applied to each sensing element at the sensed surface does not directly correspond to the portion of the load of the body of the person arranged directly above the sensing element. The load from the sensed surface that is applied to the sensing elements may be a function of the load of the body of a person resting on the support device. As such, the load of the body of a person resting of the support device may be determined from the sensed surface. The relationship between the sensed surface and the load of the body of a person resting of the support device may be dependent on the support device. As such, the electric circuitry may be calibrated for each support device to determine the specific relationship between the load of the body of a person resting on the support device and the sensed surface.

Providing the sensing elements in a single sensor pad enables straightforward insertion and removal of the sensing elements from the support device. This is advantageous for maintenance and replacement of the sensing elements. The sensor pad arrangement also enables the sensing elements to be retrofitted to existing support devices.

Arranging the sensor pad below the inflatable layer of the support device may further reduce the impact of the sensing elements on a person resting on the support device. This may further improve the comfort of a person resting on the support device and reduce the risk of formation and development of bedsores.

The sensor pad may comprise any suitable number of sensing elements. The sensor pad may comprise three, four, five or more sensing elements. The sensor pad may comprise at least three sensing elements. The sensor pad may comprise seven sensing elements. Advantageously, seven sensing elements may provide a suitable degree of precision for determining the position on the support device of a person resting on the support device, at a relatively low cost compared to larger arrays of sensing elements.

The sensing elements may be separated from one another in a direction along the width of the support device. The sensing elements may be arranged in a row extending in a direction along the width of the support device. In other words, all of the sensing elements of the sensor pad may be spaced in a line extending substantially in a direction along the width of the support device.

As used herein the term "width" is used to describe the dimension in the transverse direction of the support device and the term "length" is used to mean the dimension in the longitudinal direction of the support device. Similarly, the term "in a direction along the width of the support device" is used to mean in a direction along the transverse axis of the support device, and the term "in a direction along the length of the support device" is used to mean in a direction along the longitudinal axis of the support device. For example, where the support device comprises a mattress, the length of the mattress extends between the head end and the foot end of the mattress and the width of the mattress is the dimension perpendicular to the length, and extends between the sides of the mattress.

The sensing elements may be arranged on a board or a member to form the sensor pad. The board or member may be flexible such that it may adapt to variations in the shape of the support device.

Neighbouring sensing elements may be joined together to form the sensor pad. This arrangement may enable the sensor pad to be flexible, even if the sensing elements themselves are not flexible. This may also maintain the spatial relationship between the sensing elements. In other words, attaching together neighbouring sensing elements may maintain the spacing between the sensing elements. This may improve the accuracy and reliability of the position determination. This may facilitate insertion and removal of the sensing elements below the inflatable layer, such as for repair and maintenance.

Neighbouring sensing elements may be joined together by any suitable means. Neighbouring sensing elements may be directly attached. For example, neighbouring sensing elements may be sewn together, may be fixed together using suitable fixings, or may be bonded together using an adhesive material. Neighbouring sensing elements may be indirectly attached. For example, each sensing element may be contained within an individual envelope of material, and neighbouring envelopes of material may be attached together. For example, all of the sensing elements may be contained within a single envelope of material, and neighbouring sensing elements in the envelope may be separated by spacers or by joins between an upper surface and a lower surface of the envelope.

The sensing elements may be arranged at any suitable position relative to the inflatable layer for sensing the load exerted by a person resting on the support device.

The support device may comprise a mattress comprising the inflatable layer. The mattress may comprise more than one inflatable layer. The mattress may comprise one or more mattress underlays positioned below the inflatable layer. The mattress underlays may be fluidly interconnected to at least part of a region of the inflatable layer comprising inflatable cells. The mattress may comprise one or more support layers that are more rigid than the inflatable layer. The one or more support layers may comprise a layer of closed cell foam. The density of the one more support layers may be greater than 50 kg/m3. The mattress may comprise a coverlet to maintain the one or more layers within an interior region of the mattress. The mattress may comprise a top coverlet and a bottom coverlet. The top coverlet and the bottom coverlet may be coupled together by any suitable couplers, such as one or more zippers. An exemplary mattress system suitable for the present invention is available commercially and is marketed as the ClinActiv™ Therapy Mattress System from Hill-Rom Company, Inc.

The sensing elements may be arranged within the mattress. In other words, each sensing element may be arranged at least partially within an interior region of the mattress. This may help to maintain the position of the sensing elements relative to the mattress. This may enable the support device to be moved and transported easily.

The sensing elements may be arranged directly below the inflatable layer comprising inflatable cells. The sensing elements may be arranged below the inflatable layer and below one or more of: other layers of inflatable cells; and underlays. The sensing elements may be arranged on one of the support layers.

The sensor pad may be arranged below the mattress. In other words, the sensor pad may be arranged underneath the mattress. The sensor pad may be integral with or attached to a lower surface of the mattress. The sensor pad may be arranged outside the interior region of the mattress. This arrangement may further separate a person resting on the support device from the sensing elements and improve the comfort of the person resting on the support device.

The sensing elements may be dimensioned and arranged to substantially cover the section of the mattress corresponding to the position on the support device of the sacral region of a person resting on the support device.

Each sensing element may have a length of between about 400 mm and about 1200 mm, or between about 600 mm and about 1000 mm. Each sensing element may have a width of between about 40 mm and about 400 mm, or between about 50 mm and about 300 mm.

Each sensing element may be elongate. In other words, the length of each sensing element may be greater than the width of each sensing element. The sensing elements may be arranged with their length extending substantially in a direction along the length of the support device.

The sensing elements may be dimensioned and arranged to achieve substantial coverage of the width of the support device. For example, a typical mattress having a width of about 800 mm may have a sensor pad comprising seven sensing element, and each sensing element may have a width of about 90 mm and neighbouring sensing elements may be spaced by about 20 mm in a direction along the width of the support device.

The shape and size of each sensing element may be the same. The shape and size of each sensing element may be different. For example, the width of the sensing elements may be greater towards the sides of the support device, and smaller towards the middle of the support device. For example, the outermost sensing elements, arranged towards either side of the support device, may have a width of between about 300 mm and about 150 mm, and the innermost sensing elements, arranged towards the middle of the support device, may have a width between about 50 mm and about 120 mm. This arrangement may enable the sensing elements to extend further across the width of the support device without significantly increasing the cost of the sensor pad. This may improve the accuracy of the exit determination.

It is envisioned that each sensing element may comprise any suitable type of sensor capable of sensing a load exerted on the inflatable layer, above the sensor. For example, each sensing element may be selected from the group consisting of the following types of load cell sensing elements: strain gauge sensing elements, extensometers, bending beam sensing elements, hall-effect sensing elements and/or capacitive sensing elements.

In the present invention, the sensing elements are capacitive sensing elements. Each capacitive sensing element may comprise a flat condenser comprising: two layers of conductive material; and at least one layer of a compressible dielectric insulating material arranged between the two layers of conductive material. In particular, the capacitive sensing elements may be sensing elements of the sort described in patent US 8598893. This type of sensor may provide accurate load measurements when arranged below at least an inflatable layer comprising inflatable cells.

The layers of conductive material may comprise sheets of fabric comprising, at least partially, metallic threads of a non-oxidizing metal. The conductive sheets may comprise woven material made from threads of nickel and threads of a plastic that is at least one of polyester and polypropylene.

The dielectric insulating material may be a compressible material, and the thickness of the dielectric material may vary according to the load force or pressure applied to the condenser. The layer of compressible dielectric insulating material may be compressible in such a way that its thickness varies relative to the load applied to its surface. A variation in the thickness of the dielectric material may induce a variation in the electrical capacitance of the condenser. The layer of compressible dielectric insulating material may comprise a sheet of a synthetic elastomer with a Shore hardness of about 45 Sh to about 55 Sh. The layer of compressible dielectric material may have a thickness of between about 0.3 mm to about 1 mm.

The load or pressure sensitive element of the condenser is the layer of dielectric material, which is composed of a compressible material. It is desirable that the capacitance of the condenser is stable over time (within acceptable drift limits) and is stabilized rapidly (in a few seconds of a change of load). It is desirable that the variation in capacitance of the condenser is due solely to the variation of load or pressure applied to the condenser. The desired characteristics of the dielectric material have been determined by numerous tests, and a compromise between sensitivity and stability in terms of the thickness and the hardness of the compressible material has been found to be necessary.

The at least one layer of a compressible dielectric insulating material may be interposed between two layers of conductive material. The at least one layer of compressible dielectric insulating material and the two layers of conductive material may be applied directly against each other without interposed connecting layers. The at least one layer of compressible dielectric insulating material and the two layers of conductive material may be solidly connected to each other, for example, by tack welds that are capable of keeping the at least one layer of compressible dielectric insulating material and the two layers of conductive material in a superimposed position relative to each other.

The at least one layer of compressible dielectric insulating material and the two layers of conductive material have the same shape. The at least one layer of compressible dielectric insulating material and the two layers of conductive material have the same rectangular shape of between about 400 and 1200 mm long and between about 50 mm and about 300 mm wide. The total thickness of the at least one layer of compressible dielectric insulating material and the two layers of conductive material when superimposed together may be about 10 mm or less.

Neighbouring capacitive sensing elements may be attached to form the sensor pad. The sensor pad may comprise a plurality of elongate, flat condensers in a side-by-side arrangement. Neighbouring condensers may be joined together directly. Neighbouring condensers may be jointed together indirectly.

Neighbouring condensers may be electrically isolated from each other. For example, each condenser may be contained within an envelope of electrically insulating material. Each envelope of electrically insulating material may be attached to form the capacitive sensing pad. For example, more than one condenser may be provided in an envelope of electrically insulating material, and neighbouring condensers in an envelope may be physically and electrically separated by joins between an upper surface and a lower surface of the envelope. All of the condensers may be contained within an envelope of electrically insulating material and physically and electrically separated by separators or by joins between the upper surface and the lower surface of the envelope.

Each capacitive cell may be integrated in an oscillator capable of generating a periodic electric signal in the capacitive cell.

The frequency of the periodic electric signal generated in the capacitive cell may vary as a function of the load or pressure applied to the capacitive cell. The frequency of the periodic electric signal may decrease as the load applied to the capacitive sensing element increases. The frequency of the periodic electric signal generated in the capacitive cell may vary with weight applied to the surface of the condenser at a rate of at least 45 Hz/kg for a weight of 0 to 80 kg.

The oscillator may be any suitable oscillator. For example, the oscillator may be of astable multivibration type. An example of a suitable oscillator of astable multivibration type is a timer circuit, such as a 555 timer circuit, for example, the LMC555 CMOS timer circuit marketed by Texas Instruments, Inc.

The oscillator may have an output generating a signal comprising information related to the capacitance of the condenser. An astable multivibration oscillator may generate a rectangular wave signal at the output, with a frequency that varies as a function of the capacitance of the capacitive sensing element. The frequency of the output of the oscillator may comprise capacitance information.

The output signal of the oscillator may be directly sampled by the electric circuitry. For example, a microcontroller may be arranged to count the number of pulses of the output signal within a given period to calculate frequency. In this arrangement, an analogue-to-digital converter (ADC) is not required. The electric circuitry may be configured to determine the load applied to the capacitive sensing element based on the frequency of the oscillator output signal.

The output signal of the oscillator may be coupled to a converter for converting the frequency of the output signal to a voltage. The electric circuitry may be arranged to periodically sample the voltage signal. The electric circuitry may be configured to determine the load applied to the capacitive sensing element based on the voltage signal.

The outputs of the oscillator circuits may be connected to a sensor interface of the electric circuitry.

The electric circuitry may comprise an arrangement of electronic components. The electric circuitry may comprise a processor and a memory. The electric circuitry may comprise one or more integrated circuits (IC) or microprocessors.

The electric circuitry may be configured to periodically sample the output of the oscillators. The duty cycle of the oscillator may be between about 100 ms and 400 ms, or about 250 ms. In other words, the sampling rate of the sensor system may be about 4 Hz. The electric circuitry may be configured to periodically sample the output of the oscillators every 250 ms.

The electric circuitry may be configured to periodically collate the load measurements from the sensing elements. The electric circuitry may be configured to collate the load measurements about every 1.25 s. Where the sampling rate of the electric circuitry is about 250 ms, and the electric circuitry is configured to collate the load measurements about every 1.25 s, the electric circuitry is configured to collate six samples from the sensor pad. The electric circuitry may be configured to process the load measurements on collation.

The electric circuitry may be configured to measure any suitable quantity of a sensing element that varies as a function of the load applied to the sensing element. For example, the electric circuitry may be configured to measure one of frequency, voltage and capacitance of the sensing element.

As used herein a "load measurement" includes any suitable measurement of a quantity of a sensing element that varies as a function of the load applied to the sensing element. In other words, the load exerted on the sensing element may be derived from a "load measurements".

The electric circuitry may be configured to filter the load measurements from the sensing elements. For example, where the sensing elements comprise a capacitive sensing element and an oscillator configured to generate a periodic electric signal, the electric circuitry may be configured to filter measurements of frequency, disregarding frequency measurements outside of an operating frequency range. The frequency of the electric signal may vary substantially linearly within the operating frequency range. The operating frequency range may be between about 10 kHz to about 30 kHz or about 11 kHz and about 23 kHz or about 11.1 kHz and about 13.0 kHz.

The electric circuitry may be configured to calibrate the load measurements from each sensing element. The electric circuitry may compare the measurements from each sensing element to a minimum load threshold. The minimum load threshold may correspond to the expected load measurement from a sensing element when there is no person resting on the support device. The minimum load threshold may vary depending on the inflation of the inflatable cells of the inflatable layer.

The electric circuitry may be configured to subtract the minimum load threshold from each measurement. Where the minimum load threshold varies depending on the inflation of the inflatable layer, subtracting the minimum load threshold from the load measurements of each sensing element may remove the dependence of the load measurements on the pressure or inflation of the inflatable layer. This may isolate the component of the measurements that varies as a function of the load of a person resting on the support device.

The electric circuitry may be configured to combine the load measurements from each sensing element for each sample. The electric circuitry may be configured to combine the load measurements from each sensing element for each sample by summing the load measurements from each sensing element for each sample. The electric circuitry may be configured to combine the load measurements from each sensing element for each sample by taking an average of the load measurements from each sensing element for each sample. The electric circuitry may be configured to combine the load measurements from each sensing element for each sample by determining the arithmetic mean of the load measurements from each sensing element for each sample.

The electric circuitry may be configured to combine the load measurements from each sensing element for each sample to determine the total load of a person resting on the support device. The electric circuitry may be configured to determine the load exerted on each sensing element and combine the determined loads applied to each sensing element to determine the total load of a person resting on the support device.

The electric circuitry may be configured to determine whether a person is resting on the support device. In particular, the electric circuitry may be configured to: sample load measurements from each sensing element; combine the load measurements from each sensing element; compare the combined load measurements to a minimum load threshold value; and determine that a person is supported on the mattress if the combined measurements exceeds the minimum load threshold value. The minimum load threshold value may be stored in a memory of the electric circuitry. The minimum load threshold value may be stored in a lookup table. The minimum load threshold value may be a predetermined value. The minimum threshold value may be determined by a caregiver for each person depending on their height and weight.

Where the sensing elements are separated in a direction along the width of the support device, the electric circuitry may be configured to determine the position on the support device of a person resting on the support device in the direction along the width of the support device, based on the load sensed by each sensing element. The electric circuitry may be configured to measure the load sensed by each sensing element and compare the load sensed by each sensing element to the loads sensed by the other sensing elements to determine the position on the support device of a patient resting on the support device.

The sensor pad comprises three sensing elements: a left sensing element arranged below a left hand portion of the inflatable layer, a right sensing element arranged below a right hand portion of the inflatable layer, and a middle sensing element arranged below a middle portion of the inflatable layer. The electric circuitry may be configured to compare the load sensed by the left sensing element to the load sensed by the right sensing element and the middle sensing element, and compare the load sensed by the middle sensing element to the load sensed by the left sensing element to determine the distribution of the person's load across the support device in the direction of the width of the mattress. The distribution of the person's load may indicate whether the person is lying on the right hand portion of the support device, the left hand portion of the support device or the middle portion of the support device. In general, the greater the number of sensing elements provided in the sensor pad, the greater the accuracy of the position determination.

The electric circuitry may also be configured to determine the orientation of a person resting on the support device. In other words, the electric circuitry may be configured to determine whether a person is lying on the support device in a prone position or on their side. The electric circuitry may be configured to determine the orientation of a person resting on the support device based on a comparison of the load sensed by each sensing element to the loads sensed by the other sensing elements.

In particular, the electric circuitry may be configured to determine the difference between neighbouring sensing elements. For example, a large difference between the load measurements of neighbouring sensing elements may indicate that a person is lying on their side. This is because the person's load is distributed over a relatively narrow area of the support device. Similarly, a small difference between neighbouring sensing elements may indicate that the person is lying in a prone position. This is because the person's load is distributed over a relatively large area of the support device.

The electric circuitry may be configured to store information regarding the position of each sensing element below the inflatable layer, for example, in a lookup table. The electric circuitry may be configured to associate the measurements from each sensing element with the position information for that sensing element.

The electric circuitry is configured to determine whether a person is in an exit position on the support device. As used herein, the term 'exit position' is used to mean a position that indicates that a patient may leave or exit the support device. For example, a person resting on the support device close to either side of the support device may be positioning themselves to exit the bed or may be in danger of accidentally exiting the bed, such as by rolling over whilst asleep. The electric circuitry may be configured to trigger an alarm if it is determined that the person resting on the support device is in an exit position, close to exiting the support device.

The sensor pad comprises three sensing elements: a left sensing element arranged below a left hand portion of the inflatable layer, a right sensing element arranged below a right hand portion of the inflatable layer, and a middle sensing element arranged below a middle portion of the inflatable layer. The electric circuitry may be configured to: compare the load sensed by the middle sensing element to the determined total load of the person resting on the support device; and determine that the person resting on the support device is in an exit position on the support device if the load sensed by the middle sensing element is less than a predetermined fraction of the determined total load.. The electric circuitry may be configured to determine that the person resting on the support device is in an exit position if the load sensed by the middle sensing element is below an exit threshold. The exit threshold may be about 20% of the determined total load, or may be about 15%, 10%, or 5% of the total determined load.

The electric circuitry may be further configured to generate an output signal to activate an alarm if the person resting on the support device is determined to be in the exit position.

The inventors of the present invention have found that large movements of a person resting on the support device, such as a rolling from one side to the other side, cause temporary fluctuations in the load sensed by the sensing elements. In some embodiments, the electric circuitry may be configured to monitor these temporary fluctuations. The electric circuitry may be configured to use these temporary fluctuations to monitor the movement or activity of a person resting on the support device, and to determine whether the patient is inactive or immobile over a period of time.

The electric circuitry may be further configured to determine activity or movement of a person resting on the support device based on variations in the loads sensed by each sensing element over time. Where the sensor pad comprises at least two sensing elements, the electric circuitry may be configured to combine the loads sensed by each sensing element. This may be referred to as determining the 'sensed surface'. By comparing the sensed surface of the sensor pad between consecutive samples, movement of a person resting on the support device may be determined based on the size of the variations in the sensed surface.

In particular, the electric circuitry may be configured to: periodically sample load measurements from each sensing element; combine the load measurements from each sensing element for each sample; determine variations between the combined load measurements over time; compare the determined variations to an activity threshold; and determine that a person resting on the support device has moved if the determined variation exceeds the activity threshold.

The activity threshold corresponds to the maximum expected difference between the sensed surface for two consecutive samples for a person resting on the support device that has not moved substantially between the samples. In other words, the activity threshold corresponds to the maximum expected difference between the combined load measurements for two consecutive samples for a still person resting on the support device. The activity threshold may be an absolute value such as a load, a frequency or a voltage value. The activity threshold may be a relative value, such as a fraction or percentage. The activity threshold may be between about 0.3% and about 3%. The activity threshold value may be about 0.6%. A threshold value of about 0.6% has been found to provide a particularly suitable threshold.

Prolonged periods of inactivity of a person resting on the support device may indicate that the person is in difficulty or is at risk of developing bedsores. The electric circuitry may be configured to monitor the length of time over which a person resting on the support device is determined to be inactive or immobile. The length of time may be indicated by the number of consecutive determinations that the person resting on the support device has not moved. For example, where the sampling rate of the electric circuitry is 4 Hz, one determination of inactivity corresponds to inactivity over a time period of 250 ms, four consecutive determinations of inactivity corresponds to inactivity over a time period of 1 s and forty consecutive determinations of inactivity corresponds to inactivity over a time period of 10 s.

In particular, the electric circuitry may be configured to: determine that the person resting on the support device has not moved if a determined variation is below the activity threshold; count the number of consecutive determinations that the person resting on the support device has not moved; compare the number of consecutive determinations that the person has not moved to an inactivity threshold; and determine that the person resting on the support device is inactive if the count exceeds the inactivity threshold.

It will be appreciated that the electric circuitry may be configured to determine the period of time over which it is determined that the patient has not moved and this determined period of time may be compared to an inactivity threshold, instead of the count of the number of determinations.

The electric circuitry may also be configured to generate an output signal to activate an alarm when the person resting on the support device is determined to be inactive.

The sensor pad of the present invention, placed under the inflatable layer in the sacral region of a person resting on the mattress, enables the ideal inflation pressure of the air in the cells of the mattress to be determined, substantially as described in US 8598893. However, where the sensor pad comprises more than one sensing element, the electric circuitry may be configured to combine the measurements from each sensing element to determine at least one of the total load of the person resting on the support device and the sensed surface. The electric circuitry may be configured to use at least one of the determined total load and the sensed surface to control the inflation of the cells of the mattress.

The interface pressure exerted by the mattress on the body is directly dependent on the pressure of the air in the air-filled cells. To achieve an ideal load bearing, in other words, the lowest possible interface pressures, it is necessary to increase the patient's contact surface area with the support, hence to envelope the patient as much as possible with the support; the best support being an immersion in a liquid or pseudo liquid. However, creation of an excessive indentation must be avoided, as the patients being treated do not necessarily have good muscle tone and tend to let themselves "sink" into the mattress, which can generate undesirable stresses, notably on the thoracic cage. Ideal air pressures are thus determined experimentally by manually varying the pressure in the air cells in order to achieve the ideal load bearing relative to a person's weight, which can be correlated to the load measured by the sensor pad. As the weight distribution among the different body parts is appreciably the same for all persons, with a sensor pad in a given body region, preferably the sacral region, it is in fact possible to control an ideal inflation of the mattress relative to the various parts of the body based on the load linked to the person's weight. For a given mattress type, experimental measurements performed with patients of different weights, notably from 45 to 125 kg, make it possible to construct graphs of ideal pressures of air cells in relation to the patient's weight by adjusting the pressure of the air cells in order to achieve an ideal load bearing (minimum interface pressures and the largest possible contact zones), substantially as described in US 8598893.

The support device may further comprise a control unit. The control unit may comprise the inflation and deflation means for filling or emptying, respectively, the inflatable cells of the inflatable layer of the present invention. In other words, the control unit may comprise a source of pneumatic pressure operable to inflate the at least one inflatable bladder. The control unit may comprise electric circuitry of the present invention. The control unit may comprise the electric circuitry configured to control the inflation and deflation means for filling or emptying, respectively, the inflatable cells of the inflatable layer in such a way that the internal air pressure of the inflatable cells is a function of the load of the body of a person resting on the support device as sensed by the or each sensing element.

The control unit may comprise a main housing. The main housing may carry the source of pneumatic pressure.

The control unit may be configured to operate the support device in one or more operational modes. The control unit may be configured to control inflation of the inflatable layer depending upon the operational mode. The one or more operation modes may include an alternating pressure mode, a continuous low pressure mode and a rotation therapy mode.

It will be appreciated that other sensors may also be provided in the support device for monitoring other aspects of a person resting on the support device. For example, where the support device comprises a mattress, one or more tilt sensing elements, such as accelerometers, may be provided at least at the head zone of the mattress for sensing the angle of the head of the mattress. The angle of the head of the mattress may be used to determine the position of the patient on the mattress in the direction of the length of the mattress.

A sensor system for a support device according to the first aspect of the present invention may comprise the sensor pad and the electric circuitry. The sensor system may be removably connected to a support device. The sensor system may be retrofitted to existing support devices.

A motion monitor device for monitoring the movement of a person resting on a support device for supporting the body of a person is also disclosed, the motion monitor comprising: a sensor pad comprising at least two sensing elements separated from one another, each sensing element being configured to sense the load applied to the sensing element; and electric circuitry connected to each sensing element of the sensor pad. The electric circuitry is configured to: periodically sample load measurements from each sensing element; combine the load measurements from each sensing element for each sample; determine variations between the combined measurements over time; compare the determined variations to an activity threshold; and determine that a person supported on the support device has moved if the determined variation exceeds the activity threshold.

The electric circuitry may be further configured to: determine that the person supported on the support device has not moved if the determined variation is below the activity threshold; count the number of consecutive determinations that the person supported on support device has not moved; compare the number of consecutive determinations that the person has not moved to an inactivity threshold; and determine that the person supported on the support device is inactive if the count exceeds the inactivity threshold.

Again, it will be appreciated that the period of time over which the person is determined not to have moved may be determined by the electric circuitry and compared to the inactivity threshold.

The electric circuitry may be further configured to generate an output to activate an alarm when the person supported on the support device is determined to be inactive or immobile.

A method of determining movement or activity of a person resting on a support device may also be provided. The support device may comprise: a sensor pad comprising at least two sensing elements separated from one another, each sensing element being configured to sense the load applied to the sensing element; and electric circuitry connected to each sensing element of the sensor pad. The method may comprise the electric circuitry: periodically sampling load measurements from each sensing element; combining the load measurements from each sensing element for each sample; determining variations between the combined measurements over time; comparing the determined variations to an activity threshold; and determining that a person supported on the support device has moved if the determined variation exceeds the activity threshold.

A method of determining inactivity or immobility of a person resting on a support device may also be provided. The support device may comprise: a sensor pad comprising at least two sensing elements separated from one another, each sensing element being configured to sense the load applied to the sensing element; and electric circuitry connected to each sensing element of the sensor pad. The method may comprise the electric circuitry: periodically sampling load measurements from each sensing element; combining the load measurements from each sensing element for each sample; determining variations between the combined measurements over time; comparing the determined variations to an activity threshold; determining that the person supported on the support device has not moved if the determined variation is below the activity threshold; counting the number of consecutive determinations that the person supported on support device has not moved; comparing the number of consecutive determinations that the person has not moved to an inactivity threshold; and determining that the person supported on the support device is inactive if the count exceeds the inactivity threshold.

The invention will now be further described by way of example only and with reference to the accompanying figures in which:
Figure 1 is a schematic illustration of an exemplary mattress system for use with embodiments of the present invention;
Figure 2 is a schematic illustration of a sensor system of the present invention, the sensor system comprising a sensor pad and electric circuitry according to the present invention;
Figure 3 is a schematic illustration of a capacitive sensing element for use in the sensor pad of Figure 2;
Figure 4 is a schematic illustration of an oscillator circuit for the capacitive sensing element of Figure 3;
Figure 5 is a flow diagram showing exemplary steps performed by electric circuitry of Figure 2;
Figure 6 is a flow diagram showing exemplary steps performed by the electric circuitry of Figure 2 to determine whether a person resting on a support device is in an exit position or has exited the support device according to the present invention;
Figures 7a, 7b and 7c are histograms showing exemplary load measurements from the sensor system of Figure 2, with a person resting on a support device, above the sensor pad, in different positions on the support device and in different orientations;
Figure 8 is a flow diagram showing exemplary steps performed by the electric circuitry of Figure 2 to determine whether a person resting on a support device is about to exit the support device according to the present invention;
Figure 9 is a flow diagram showing exemplary steps performed by the electric circuitry of Figure 2 to determine the mobility or activity of a person resting on a support device according to the present invention;
Figure 10 is a graph showing exemplary fluctuations in load measurements of the sensor pad of Figure 2, caused by movements or activity of a person supported on a support device according to the present invention; and
Figure 11 is a flow diagram showing exemplary steps performed by the electric circuitry of Figure 2 to determine the inflation of an inflatable layer of a support device according to the present invention.

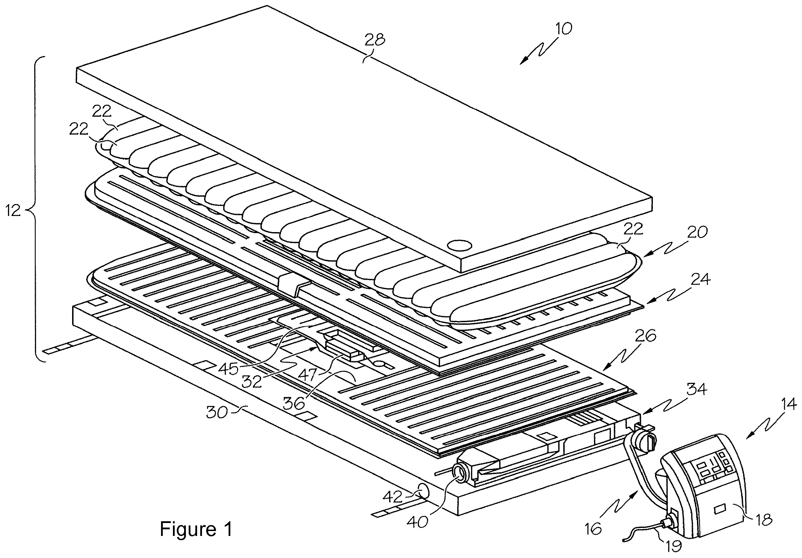

An exemplary support device suitable for the present invention may is provided in a mattress system available commercially and is marketed as the ClinActiv™ Therapy Mattress System from Hill-Rom Company, Inc. A schematic illustration of such an exemplary mattress is shown in Figure 1, which is taken from United States patent no. US 7849545. The mattress system 10 shown in Figure 1 comprises a mattress 12 and a control unit 14. The control unit 14 is spaced-apart from the mattress 12 and is coupled pneumatically and electrically with the mattress 12 by a connector assembly 16. The control unit 14 includes a plurality of user interface modules (not shown), which may be selectively coupled to the control unit 14 to configure the mattress system 10 with various functionalities. For example, one module may enable the mattress to be operated in a continuous low pressure mode and another module may enable the mattress to be operated in an alternating pressure therapy mode.

As shown in Figure 1, the exemplary mattress 12 includes an upper inflatable bladder layer 20 having a plurality of laterally-extending inflatable cells or bladders 22. The plurality of laterally-extending cells or bladders 22 cooperate to define various zones of layer 20. For example, a head section zone comprises the first three cells 22 of layer 20, a torso section zone comprises the next ten cells 22 of layer 20 and a heel section zone comprises the last seven cells 22 of layer 20. The cells 22 of the head section zone are fluidly interconnected and regulated to the same pressure. The cells 22 of the torso section zone are grouped into a first group of five cells and a second group of five cells. The cells of the first group of five cells are fluidly interconnected and regulated to the same pressure, and the cells of the second group of five cells are fluidly interconnected and regulated to the same pressure. The cells 22 of the heel section zone are also fluidly interconnected and regulated to the same pressure. The pressure of the cells 22 may be regulated by electronics within the control unit 14. In particular, the pressure of the cells 22 of the heel section zone may be regulated to a target pressure that is independent of the pressure of the head section zone and the torso section zone. The head, torso and heel section zones may be controlled and operated independently of each other by the control unit 14.

The mattress 12 also comprises a first air mattress underlay 24, positioned below the layer of cells 20, and a second mattress underlay 26, positioned below the first air mattress underlay 24. The first and second mattress underlays 24, 26 are fluidly interconnected and regulated to the same pressure as the head section zone of the layer 20. The mattress 12 further includes a top coverlet 28 and a bottom coverlet 30. The top coverlet 28 and the bottom coverlet 30 are coupled together by suitable couplers, such as one or more zippers, to maintain the layer 20 and underlays 24, 26 within an interior region of the mattress 12.

The mattress 12 further comprises a pressure sensor 32 and a technical box 34. The technical box 34 comprises an air distribution system (not shown), which includes: a set of valves, associated circuitry, and a manifold assembly. The manifold assembly is in fluid communication with various cells 22 of the layer 20 and the first and second air mattress underlays 24, 26. The manifold assembly includes a pneumatic input and a plurality of pneumatic outputs, each of which is associated with its own valve of the set of valves. The control unit 14 may control the position of the valves which may control the inflation and deflation of the zones in accordance with the desired mode of operation of the mattress.

As shown in Figure 1, the technical box 34 is positioned at a foot end of the mattress 12, proximal to the foot section zone of layer 20 such that foot ends of the air mattress underlays 24, 26 generally abut the technical box 34 but do not extend over the top of the technical box 34. However, the layer 20 (and particularly the heel zone bladders 22 of the layer 20) extends over the technical box 34. When the mattress 12 is assembled, the pressure sensor 32 of the mattress 12 is positioned generally below the torso section zone of the layer 20 in order to sense the pressure exerted by a person on the torso section zone and provide an output signal to the control unit 14 indicating the sensed pressure.

The pressure sensor 32 is positioned within a cut-out section 36 formed in the second air mattress underlay 26. When the mattress 12 is assembled, the pressure sensor 32 may be positioned generally below the torso section zone of the layer 20 in order to sense the pressure exerted by a person on the torso section zone. When a person is supported on the mattress 12, the person's torso applies pressure on the mattress 12, which is transferred as a force to the pressure sensor 32. The pressure is sensed by the pressure sensor 32 and the pressure sensor 32 provides an output signal to the control unit 14 indicating the sensed pressure.

The data from pressure sensor 32 is used in three distinct operation modes of system 10 including stand-by regulation, person egress surveillance, and main zone pressure regulation. The stand-by regulation mode means that system 10 operates to achieve a relatively low pressure in the mattress 12, so as to provide a ready-to-use, and yet comfortable, support when the person is initially placed on the mattress 12. The person egress surveillance mode means that system 10 operates to monitor the presence and absence of the person on the mattress 12. The main zone pressure regulation mode means that system 10 operates to control the internal pressures in the bladders 22 of layer 20 and underlays 24, 26. Illustratively, the circuitry 47 of pressure sensor 32 is in electrical communication with the circuitry (not shown) within the technical box 34. As noted above, the circuitry within the technical box 34 is in electrical communication with the control unit 14 via the connector assembly 16.

In some embodiments, one or more additional sensing elements are provided in technical box 34 and are associated with one or more of the pneumatic lines used to inflate layer 20 and underlays 24, 26. The additional sensing elements may detect and communicate the pressure within one or more of layer 20, each zone of layer 20 and underlays 24, 26 to the control unit 14.

Mattress 12 also includes a cardiopulmonary resuscitation (CPR) assembly that is coupled to the technical box 34 and that includes a rotatable knob 40 that is accessible to the caregiver through an aperture 42 formed in the foot end portion of the bottom coverlet 30.

The sensor pad of the present invention comprises a plurality of sensing elements arranged below the inflatable layer. The sensor pad may be arranged in a similar position in the mattress as the pressure sensor 32, described in United States patent no. US 7849545. In other words, the sensor pad may be positioned within a cut-out section 36 formed in the second air mattress underlay 26 and when the mattress is assembled, the sensor pad may be positioned generally below the torso section zone of the inflatable layer of cells in order to sense the load exerted by a person on the torso section zone. The sensing elements of the sensor pad are typically spaced in a direction along the width of the mattress.

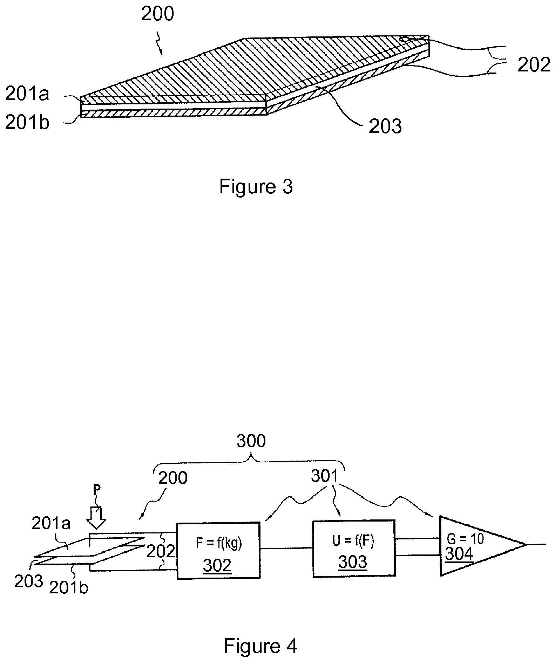

The pressure sensor 32 described in United States patent no. US 7849545 comprises a single liquid-containing flexible enclosure 45 and associated circuitry. Although each sensing element of the sensor pad of the present invention may comprise the type of sensor described in United States patent no. US 7849545, preferably, each sensing element comprises a capacitive load sensor, such as the sensing elements described in United States patent no. US 8598893, which are shown in Figures 3 and 4 and described in further detail below.

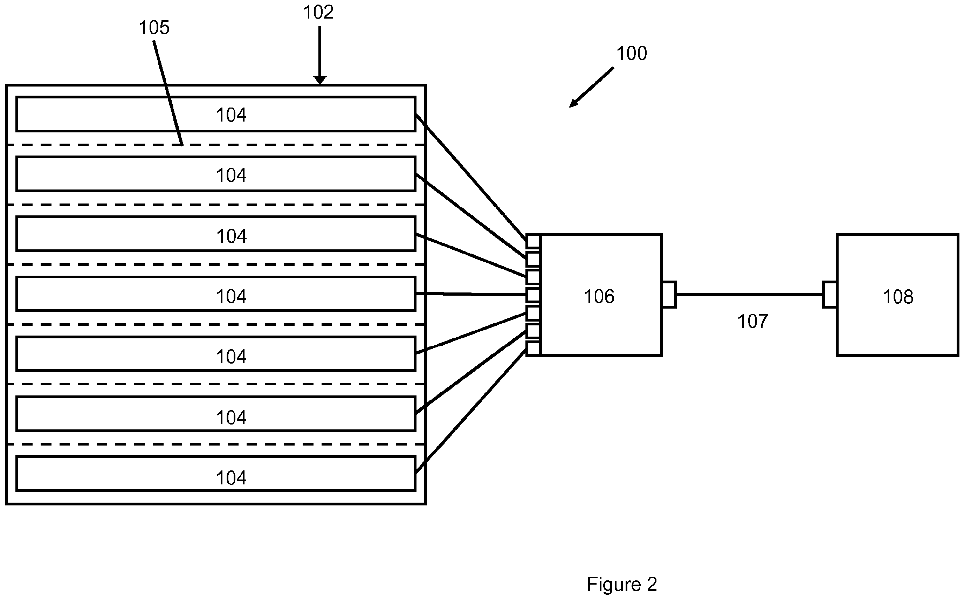

A sensor system according to the present invention is shown in Figure 2. The sensor system 100 comprises a sensor pad 102 comprising seven load sensing elements 104. Each sensing element 104 is elongate and arranged side-by-side with the other sensing elements 104 in a row. Neighbouring sensing elements 104 are joined at abutting sides 105 to form a single unit, which is referred to herein as the sensor pad 102. Each sensing element 104 comprises an output which is connected to a sensor interface board 106. The sensor interface board 106 may comprise a microcontroller such as the MSP430F247TPM microcontroller available from Texas Instruments, Inc. The microcontroller may be programmed to process measurements received from the sensing elements and perform the functions of the electric circuitry of the present invention. The sensor interface board 106 comprises an output 107 which is connected to the control unit 108 of the mattress.

In use, the sensor pad 102 may be arranged below an inflatable layer of inflatable cells of a mattress below the region of the mattress that supports the sacral region of a person resting on the support device. Placement of the sensor pad 102 under the inflatable layer at a position corresponding to the person's sacrum is desirable because the sacrum is the part of the body that projects the most when the patient is reclining on his back and it is also the region on which a patient rests when in a sitting position and that exerts the greatest load. Preference is also given to placement of the sensor pad in the sacral region because as a general rule, the hinged frames on which the mattresses are typically supported are immovable in this region.

The sensor pad 102 may be arranged with the length of each sensing element 102 aligned with the length of the mattress and the sensing elements being spaced in a direction along the width of the mattress. In other words, the sensing elements are arranged laterally across the mattress such that some of the sensing elements are arranged to sense loads supported on the left hand side of the mattress, some of the sensing elements are arranged to sense loads supported in the centre of the mattress and some of the sensing elements are arranged to sense loads supported on the right hand side of the mattress. This arrangement may enable the sensor pad 102 to sense movements of a person across the mattress between the left and right hand sides of the mattress.

The interface board 106 may be arranged in the mattress with the sensor pad; however, preferably the interface board 106 is arranged outside of the mattress. The interface board 106 may be housed with the control unit 108 of the mattress or may be separate of the control unit 108. The sensor interface board 106 may comprise all of the electric circuitry of the electric circuitry of the present invention, or the electric circuitry of the electric circuitry may be split between the sensor interface board 106 and the control unit 108.

Preferably, the sensing elements 102 of the sensor pad 104 are capacitive sensing elements. A capacitive load sensor suitable for use in the present invention is described in United States patent no. US 8598893. Schematic illustrations of a suitable, exemplary capacitive load sensor and an exemplary sensor oscillator circuit comprising the capacitive sensor are shown in Figures 3 and 4 respectively, which are both taken from United States patent no. US 8598893.

Figure 3 shows a capacitive sensing element comprising a flat condenser 200 including a stack of three planar layers with the same rectangular dimensions. The three layers include a layer of dielectric insulating material 203 interposed between two conductive layers 201a, 201b, comprising sheets of conductive material.

The middle layer 203 of dielectric insulation material is compressible in such a way that its thickness varies relative to the load applied to its surface. The thickness of the dielectric varies according to the load force or pressure applied to the condenser 200, which in turn induces a variation in the electric capacitance of the condenser 200. This variation in capacitance may be measured and used to determine the load applied to the condenser 200.

The condenser 200 is integrated in an oscillator circuit 300, which further comprises circuitry 301. The circuitry 301 comprises an oscillator of astable multivibration type 302, which generates a periodic rectangular output signal. The frequency of the output signal varies with the capacitance of the condenser 200. In the embodiment shown in Figure 4, the circuitry 301 further comprises means for processing the output signal from the oscillator 302 before the output signal is received by the electric circuitry. The means for processing the output signal from the oscillator comprises a frequency/voltage converter 303 and a differential amplifier 304.

It will be appreciated that the circuitry 301 may not comprise means for processing the signal from the oscillator and instead the electric circuitry may receive the output from the oscillator 302 directly. Since the frequency of the output signal from the oscillator relates the capacitance of the condenser 200, the electric circuitry may directly use the frequency of the output signal as an indication of the load applied to the condenser 200.

The connection of the conductive sheets 201a, 201b to the circuitry 301 is achieved by connective wires 202 equipped with press buttons on their ends that are identical to those used for attaching EKG electrodes. This type of metal press button is widely used in the garment industry; they are inexpensive and easy to attach. The ends of the wires for connecting the sensing element to the control device are equipped with a press button that is compatible with that of the sensing element for enabling the coupling of these components.

The final assembly of the three layers of the condenser is sandwiched between two impermeable polyurethane sheets joined together by welding or by any other means in order to protect the sensing element and ensure that it is relatively well sealed.

The circuitry 301 employs a frequency/voltage conversion to the output of the oscillator 302. A convertor 303 converts the frequency (Hz) of the output signal from the oscillator 302 into a voltage signal (Volt). The amplitude of the voltage signal varies with the frequency of the input signal from the oscillator. If the frequency of the input signal increases, the amplitude of the output voltage signal increases. Hence, a signal expressed as U=f(F) is obtained, and hence U=f(d) or even U=f(kg). The voltage signal from the converter 303 is amplified by a differential amplifier 304 before being forwarded to the electric circuitry (not shown).

This assembly exhibits good response and sensitivity properties, as it is possible to distinguish a force of 1 kg for a load ranging from 0 to 80 kg applied to the sensing element, with a sensitivity greater than 50 mV/kg by using a dielectric layer composed of a compressible insulating material exhibiting the thickness and hardness characteristics defined below.

The frequency of the oscillator varies from 15 kHz to 18 kHz for applied loads as great as 80 kg, which is above the audible frequencies that could generate an annoying whistling, and below the frequencies that could interfere with the operation of another nearby electronic device.

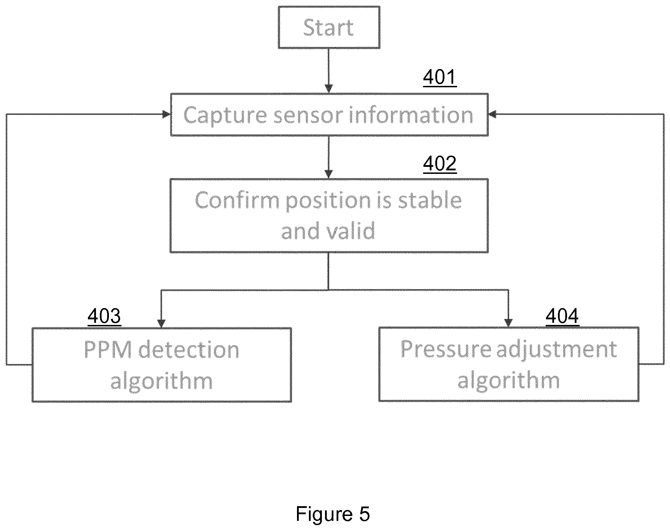

Figure 5 shows an exemplary set of steps performed by the electric circuitry of Figure 2 to determine the position on a support device of a person resting on the support device, above the sensor pad, and to determine the pressure of the inflatable cells of the inflatable layer.

At a first step 401, the electric circuitry periodically obtains load measurements from the outputs of each of the sensing elements. At a second step 402 the electric circuitry determines whether a person is resting on the support device and determines that the person is not moving on the support device. In third and fourth steps 403, 404, the electric circuitry determines the position on the support device of the person resting on the support device based on the load measurements and determines the ideal pressure of the inflatable cells of the inflatable layer based on the load measurements.

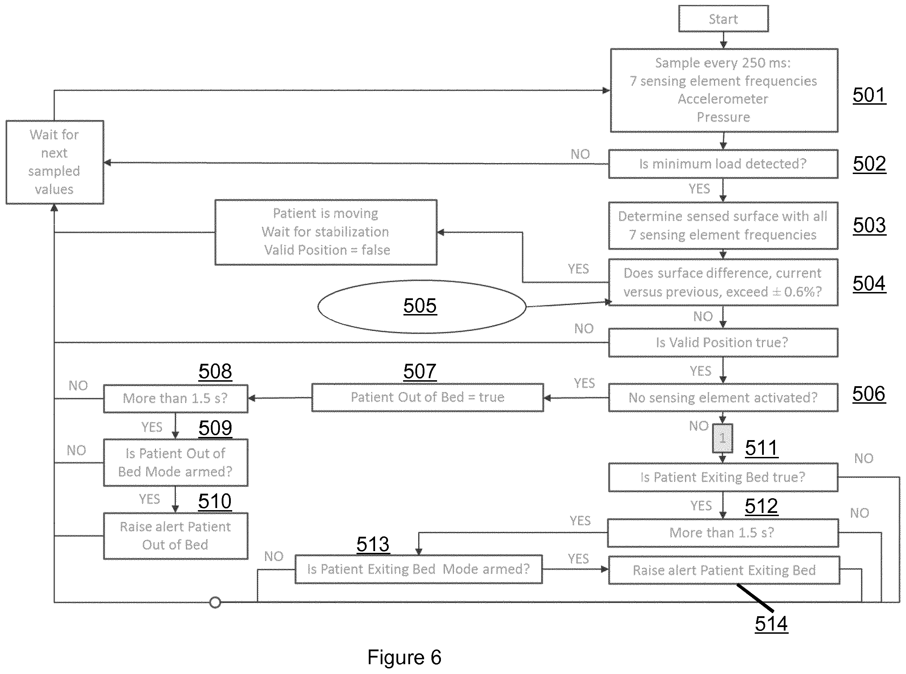

Figure 6 shows a flow diagram of an exemplary set of steps that the electric circuitry of the sensor system 100 of Figure 2 may be programmed to perform to determine the position of a person supported on a support device based on the load measurements and subsequently whether the person is in an exit position on the support device.

As shown in Figure 6, in a first step 501, the electric circuitry periodically measures the frequency of the output signals from each of the seven sensing elements. Each frequency measurement contains information about the capacitance of the sensing element, which varies with the load applied to the sensing element. The electric circuitry samples the outputs every 250 ms and collates the measurements every 1.25 s. In other words, the electric circuitry processes six samples of seven measurements every 1.25 s.

In some embodiments, the electric circuitry may determine whether the frequency measured for each sensing element for the current sample is within an operating frequency range of about 11 kHz and about 23 kHz. Frequency measurements outside of this range may be disregarded. If all seven frequency measurements for a sample are outside of the operating frequency range, the electric circuitry may perform no further processing on the measurements of the current sample and may wait for the next sample measurements.

In a second step 502, the electric circuitry combines the frequency measurements from each of the sensing elements for the current sample to provide an indication of the total load supported on the mattress. The determined total load resting on the mattress is compared to a minimum load threshold stored in the memory of the electric circuitry. The minimum load threshold corresponds to the minimum load expected if a person is supported on the mattress.

If the total load resting on the mattress is determined to be below the minimum load threshold, the electric circuitry determines that a person is not supported on the mattress and performs no further processing on the measurements from the current sample.

If the total load supported on the mattress is determined to exceed the minimum load threshold, in a third step 503, the electric circuitry determines the 'sensed surface' for the current sample. This means that the electric circuitry combines the frequency measurements from each of the sensing elements for the current sample, and calculates the arithmetic mean.

It will be appreciated that if the frequency measurements may be combined and compared directly to a minimum load threshold. In this case, the electric circuitry will be configured to determine that a person is resting on the mattress if the combined frequencies are determined to be below the minimum load threshold. This is because the frequency of the output signal of each sensing element increases as the load applied to the sensing elements decreases.

In a fourth step 504, the electric circuitry compares the sensed surface for the current sample to the sensed surface for the previous sample. In other words, the electric circuitry determines the difference between the arithmetic mean of the current sample and the arithmetic mean of the previous sample. This difference is referred to as the 'fluctuation'. The fluctuation between the sensed surfaces of the current sample and the previous sample provides an indication of whether a person supported on the support device has moved on the mattress between the previous sample and the current sample. The fluctuation may be calculated an absolute value, such as a frequency value; however, typically the fluctuation is calculated as a percentage difference between the current and the previous sample measurements.

The electric circuitry compares the determined fluctuation to a fluctuation threshold. The fluctuation threshold corresponds to the minimum fluctuation that would be expected if a person supported on the support device has moved or changed position on the mattress between the previous sample and the current sample (i.e. over a period of 250 ms). The caregiver may set the fluctuation threshold according to the type of patient and the patient's condition. However, a fluctuation threshold value of about 0.6 % has been found to be suitable for most patients.

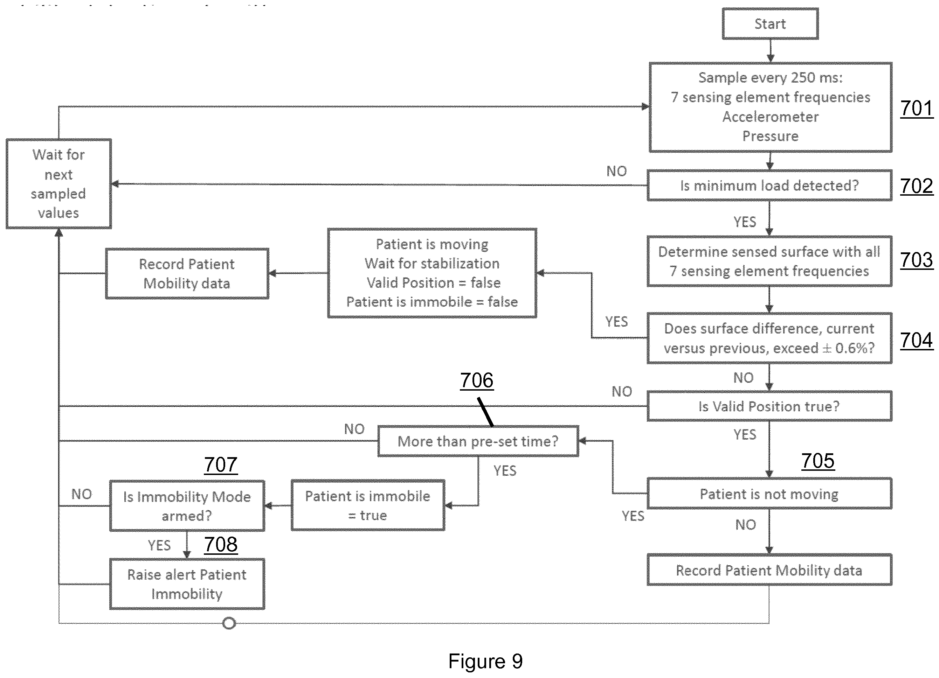

If the determined fluctuation exceeds the fluctuation threshold, the electric circuitry determines that the person resting on the support device has moved between the previous sample and the current sample. At this stage, the electric circuitry may determine 505 whether the person resting on the support device is active, as described in more detail below, in reference to Figure 9.

If the electric circuitry determines that the person has moved, the electric circuitry determines that the person is active and the person is not in a stable position. If the electric circuitry determines that the person is active and not in a stable position, the electric circuitry performs no further processing on the measurements from the current sample.

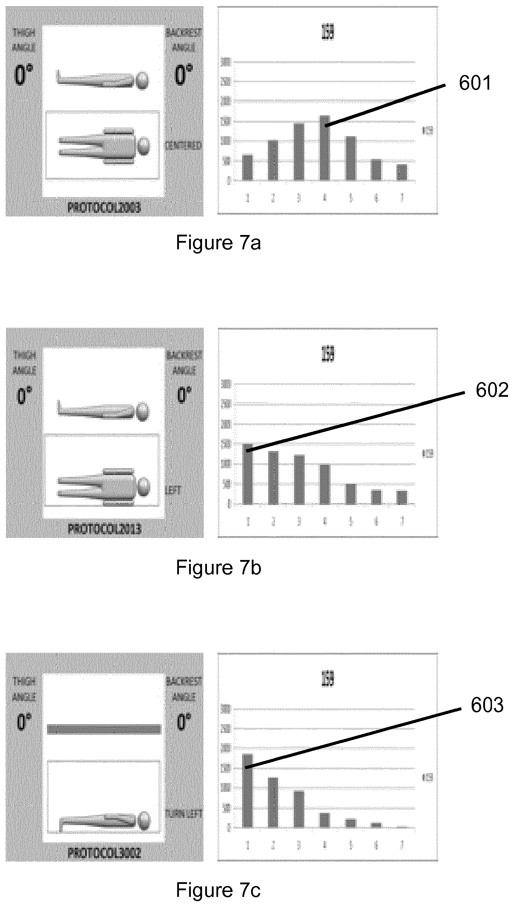

If the determined fluctuation is below the fluctuation threshold, the electric circuitry may determine the position on the support device of the person resting on the support device. Figures 7a, 7b and 7c show histograms of exemplary load measurements from the sensor pad of Figure 2 with a person resting on the support device in different positions and orientations.

Figures 7a, 7b and 7c show histograms of exemplary samples from the seven sensing elements of the sensor pad of Figure 2, arranged below an inflatable layer of a mattress. The histograms indicate how the position on the mattress and the orientation of a person resting on the mattress may be determined by comparing the load measurements of each sensing element to the measurements of the other sensing elements.

The sensor pad is arranged below the sacral region of a person resting on the mattress and the sensing elements are separated in the direction along the width of the mattress, arranged in a row. The histograms show the measured load of each sensing element for a single sample. The sensing element load measurements are shown with the leftmost sensing element (sensing element 1) on the left of the graph to the rightmost sensing element (sensing element 7) on the right of the graph, as the sensing elements are arranged below the inflatable layer.

Figure 7a shows load measurements for the seven sensing elements sampled when the person was resting in the middle of the mattress, in a prone position. The centremost sensing element shows the largest measurement 601, and the measurements of the neighbouring sensing elements decrease gradually towards the right and left hand sides of the mattress. The position of the peak load measurement at the middle sensing element indicates that the person is located in the middle of the mattress. The gradual decrease of the measurements from the centre towards the left and right hand sides of the mattress indicates that the person's load is spread out across a relatively large portion of the width of the mattress, which indicates that the person is lying in a prone position.

Figure 7b shows load measurements for the seven sensing elements sampled when the person was resting on the left hand side of the mattress, in a prone position. The leftmost sensing element shows the largest measurement 602, and the measurements of the neighbouring sensing elements decrease gradually towards the right hand side of the mattress. The position of the peak measurement at the leftmost sensing element indicates that the person is located at the left hand side of the mattress. The gradual decrease of the measurements towards the right hand side indicates that the person's load is spread out over a relatively large portion of the width of the mattress, which indicates that the person is lying in a prone position.

Figure 7c shows load measurements for the seven sensing elements sampled when the person was resting on the left hand side of the mattress, on their side. The leftmost sensing element shows the largest measurement 603, and the readings of the neighbouring sensing elements decrease much more rapidly towards the right hand side of the mattress than the measurements shown in figures 7a and 7b. The position of the peak measurement at the leftmost sensing element indicates that the person is located at the left hand side of the mattress. The rapid decrease of the measurements towards the right hand side indicates that the person's load is spread out across a relatively narrow portion of the width of the mattress, which indicates that the person is lying on their side.

Returning to Figure 6, the electric circuitry may be configured to store position information for each of the sensing elements in a lookup table. The electric circuitry may compare each load measurement from each sensing element for the current sample to the load measurements from the other sensing elements for the current sample. The electric circuitry may determines the peak load measurement and uses the stored position information to determine the position of the peak measurement in relation to the support device. This indicates the position on the support device of the person resting on the support device. The electric circuitry may also determine the difference between the measurements of neighbouring sensing elements and uses these differences to determine the orientation of the person resting on the support device.

In other embodiments, the electric circuitry may store reference histogram data in a lookup table. The reference histogram data may be associated with position and orientation information, and the electric circuitry may be configured to compare the load measurements from each sensing element to the stored histogram data and determine at least one of the position and orientation of the person resting on the support device based on a match.

If the electric circuitry is unable to determine a position of the person on the support device, the electric circuitry may determine that the position is not a true position, and the electric circuitry may perform no further processing on the measurements from the current sample. If the electric circuitry is able to determine the position of the person resting on the support device, the electric circuitry may determine the position of the person to be a true position, and continue to step 506.

If the electric circuitry determines the person to be in a stable position, the electric circuitry performs step 506. In step 506, the electric circuitry determines whether each load measurements for each of the sensing elements for the current sample is above a minimum load threshold. The minimum load threshold may be set by a caregiver depending on the size and weight of a patient to be supported by the support device.

A sensing element that generates an output signal above the minimum load threshold is referred to as being 'activated'. In other words, an 'activated' sensor is a sensor that senses a load resting above it on the support device.

If the electric circuitry determines that none of the sensing elements are 'activated' (if none of the load measurements are above the minimum load threshold), the electric circuitry determines at step 507 that the person is not resting on the support device, or is 'out of bed'.

If the electric circuitry determines that the person is 'out of bed', the electric circuitry records the out of bed determination and counts the number of previous, consecutive determinations that the patient was out of bed. This count relates to the period of time over which the person has been determined to be out of bed. For example, two consecutive determinations that the patient is out of bed corresponds to the period of time between the two samples, which in this embodiment is 250 ms.

In a further step 508, the electric circuitry compares the count of the pervious, consecutive determinations that the person is out of bed to an out of bed threshold. The out of bed threshold corresponds to the maximum acceptable period of time over which the patient may be determined to be out of bed. The out of bed threshold may be set to reduce the effect of erroneous determinations that the person is out of bed. Typically the out of bed threshold will correspond to between about 1 second and 5 seconds. In this embodiment, the out of bed threshold corresponds to 1.5 seconds, which is equivalent to 6 consecutive determinations that the patient is out of bed.

If the count of previous, consecutive determinations that the person is out of bed is below the out of bed threshold, the electric circuitry performs no further processing on the measurements from the current sample. If the count of previous, consecutive determinations that the person is out of bed exceeds the out of bed threshold, the electric circuitry checks whether an out of bed alarm is armed in a further step 509. If the out of bed alarm is not armed, the electric circuitry performs no further processing on the measurements from the current sample. If the out of bed alarm is armed, the electric circuitry sends a signal to raise an alarm that the person has exited the support device in a further step 510. The alarm may, for example, be an electronic message sent to a computer monitored by a caregiver or an audible alarm.

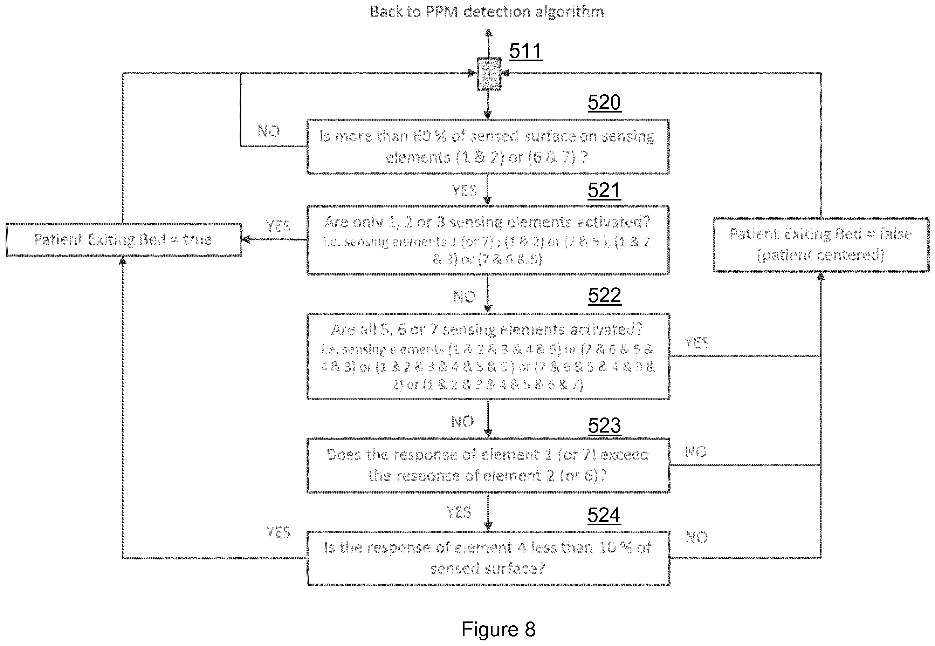

Returning to step 506, if the electric circuitry determines that one or more of the sensing elements are activated, in other words, one or more of the load measurements exceed the minimum load threshold, the electric circuitry proceeds to determine whether the person is in an exit position, as shown in Figure 8.

As shown in Figure 8, the electric circuitry is configured to determine whether the person is in an exit position on the support device. An exit position is a position in which a significant proportion of the load of the person is resting near to one of the sides of the support device. A person resting in this type of position on the support device may be close to getting out of or falling out of the support device.

The electric circuitry is configured to compare the load measurements from each of the sensing elements for the current sample with the load measurements from the other sensing elements and with the total load determined at step 502.

As mentioned above, the electric circuitry stores position information regarding each of the sensing elements. In this embodiment, the sensing elements are each provided with numbers from 1 to 7. Number 1 represents the sensing element arranged at the leftmost edge of the sensor pad, below the left hand side of the mattress, and number 7 represents the sensing element arranged at the rightmost edge of the sensor pad, below the right hand side of the mattress.

In a first step 520, electric circuitry compares the measurements from the leftmost sensing elements 1 and 2 to the total load, and the measurements from the rightmost sensing elements 6 and 7 to the total load. If the electric circuitry determines that either the measurements from the leftmost sensing elements 1 and 2 or the measurements from the rightmost sensing elements 6 and 7 do not comprise more than 60 % of the total load, the electric circuitry determines that the person is not in an exit position and does not perform any further processing on the measurements from the current sample.

If the electric circuitry determines that either the measurements from the leftmost sensing elements 1 and 2 or the measurements from the rightmost sensing elements 6 and 7 do comprise more than 60 % of the total load, the electric circuitry performs further processing 521 on the measurements from the current sample.

In a second step 521, the electric circuitry determines which of the sensing elements are activated. If the electric circuitry determines that one or more of sensing elements 1, 2 or 3 are activated and none of the other sensing elements are activated or that one or more of sensing elements 5, 6 or 7 are activated and none of the other sensing elements are activated, the electric circuitry determines that the person resting on the support device is in an exit position and proceeds to step 512, shown in Figure 6.

Turning back to Figure 6, if the electric circuitry determines that the person is in an exit position, the electric circuitry determines 512 whether the exit position has been determined consecutively for a period of 1.5 s, determines 513 whether the patient exit mode is armed and raises an exit alarm 514, similarly to steps 508, 509 and 510 in relation to the out of bed determination described above.

Turning back to Figure 8, If the electric circuitry determines that more than only sensing elements 1, 2 and 3 or only sensing elements 5, 6 and 7 are activated, the electric circuitry performs further processing step 522 on the load measurements from the current sample.

In a third step 522, the electric circuitry determines whether five, six or all seven of the sensing elements are activated. If the electric circuitry determines that five or more sensing elements are activated, the electric circuitry determines that the person is not in an exit position and performs no further processing on the measurements from the current sample.

If the electric circuitry determines that less than five sensing elements are activated, the electric circuitry determines, in a further step 523, whether the load measurement of the leftmost sensing element, sensing element 1, exceeds the load measurement of the neighbouring sensing element 2, or whether the load measurement of the rightmost sensing elements 7 exceeds the load measurement of the neighbouring sensing element 6. If the load measurement of the leftmost sensing element 1 or the rightmost sensing element 2 does not exceed the load measurement of the neighbouring sensing element, the electric circuitry performs no further processing on the measurements from the current sample.

If the load measurement of the leftmost or the rightmost sensing element exceeds the load measurement of the neighbouring sensing element, the electric circuitry performs a further processing step 524 on the measurements from the current sample.

In a fourth step 524, the electric circuitry compares the measurement of the centremost sensing element, sensing element 4, to the total load. If the electric circuitry determines that the measurement of sensing element 4 exceeds 10% of the total load, the electric circuitry determines that the person is not in an exit position and performs no further processing on the measurements from the current sample.

If the electric circuitry determines that the measurement of sensing element 4 is less than 10% of the total load, the electric circuitry determines that the person is in an exit position and proceeds to step 512, shown in Figure 6.

As shown in Figure 9, the electric circuitry may also monitor the movement or the activity of a person resting on the support device. It will be appreciated that the steps shown in Figure 9 may also be used in a motion monitoring system in accordance with the second aspect of the present invention.

Initial steps 701, 702, 703 and 704 are identical to steps 501, 502, 503 and 504 described above in relation to Figure 6. These steps may be performed by the electric circuitry at the same time. Indeed, Figure 6 shows at step 505 that the movement or activity of the person may be monitored.

If the electric circuitry determines that the fluctuation of the current sample did not exceed the fluctuation threshold, the electric circuitry determines, at step 705, that the person has not moved between the previous sample and the current sample.

If the electric circuitry determines that person has not moved between the previous sample and the current sample, the electric circuitry records the determination and counts the number of previous, consecutive determinations that the patient has not moved. This count relates the period of time over which the person has been determined to have been inactive. For example, two consecutive determinations that the patient has not moved corresponds to the period of time between two samples, which in this embodiment is 250 ms.

At step 706, the electric circuitry compares the count of the pervious consecutive determinations that the person has not moved to an inactivity threshold. The inactivity threshold corresponds to a maximum acceptable period of time over which the patient may be determined to have been inactive. Typically the period of time will be between about 10 second and 30 minutes. In this embodiment, the acceptable time period is 20 seconds, which corresponds to 80 consecutive determinations that the patient has not moved.

If the count of previous, consecutive determinations that the person has not moved is below the inactivity threshold, the electric circuitry performs no further processing on the measurements from the current sample.

If the count of previous, consecutive determinations that the person has not moved exceeds the inactivity threshold, the electric circuitry checks 707 whether an inactivity alarm is armed. If the inactivity alarm is not armed, the electric circuitry performs no further processing on the measurements from the current sample. If the inactivity alarm is armed, the electric circuitry proceeds to send 708 a signal to raise an alarm. The electric circuitry may be configured to raise the alarm by, for example, sending an electronic message to a caregiver, by issuing an audible alarm or by issuing a visual alarm, such as by illuminating an LED on the control unit.

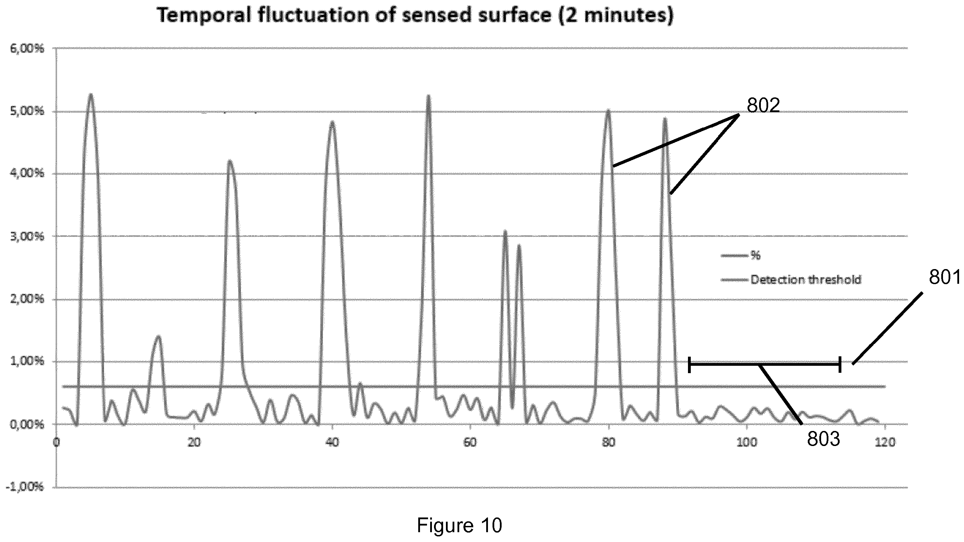

Figure 10 shows a graph of exemplary fluctuations measured over a period of time. The graph illustrates the minimum fluctuation threshold 801, which in this example is 0.6 %. A series of peaks 802 exceed the threshold 801. Each peak 802 corresponds to a movement of a person resting on the support device.

Prolonged periods of time over which no movement of the person is detected, such as the period 803, may indicate that the person is incapacitated or immobile. The electric circuitry may be configured to monitor for such prolonged periods of time and to raise an alarm if the patient has not moved for a predetermined period of time.

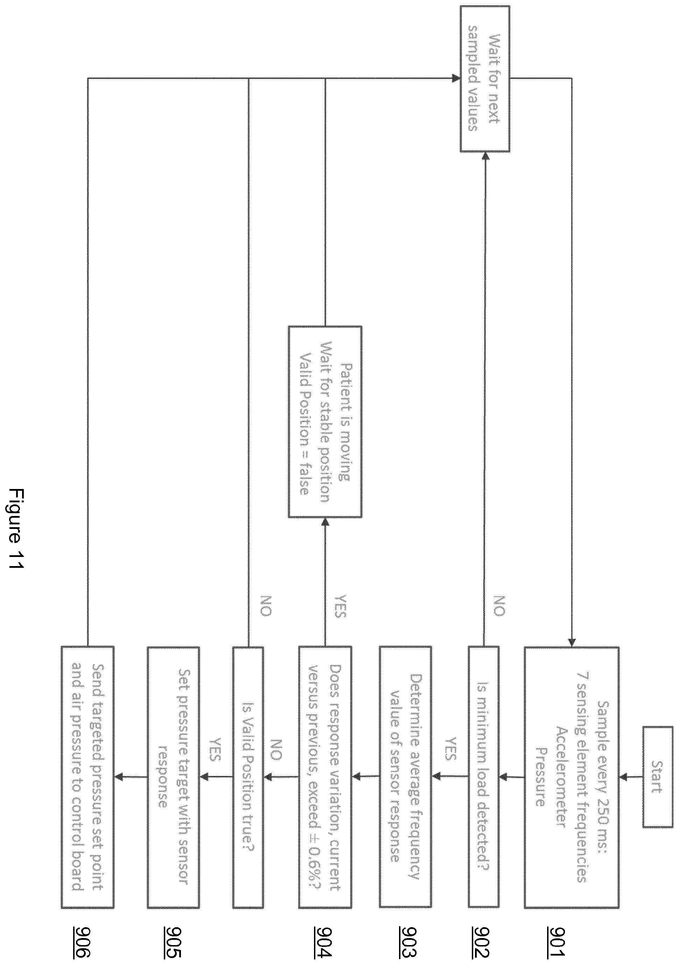

Figure 11 shows a flow diagram of an exemplary set of steps that the electric circuitry of the sensor system 100 of Figure 2 may be programmed to perform to determine the pressure of the inflatable cells of the inflatable layer.

Steps 901, 902, 903 and 904 are identical to steps 501, 502, 503 and 504 described above in relation to Figure 6. These steps may be performed by the electric circuitry at the same time.

If the determined fluctuation for the current sample does not exceed the fluctuation threshold, the electric circuitry sets 905 a set point or a target pressure for the inflatable cells based on the sensed surface. In other words, based on the load measurements taken by the electric circuitry, the pressure of the air cells can be automatically regulated relative to a set point that may be determined by experiment, as described in US 8598893. The electric circuitry calculates the correct set point for regulating the air pressure in the mattress cells as a function of the data generated by the sensor pad. Once the electric circuitry has determined the set point, the electric circuitry sends 906 to set point to the inflation and deflation means to inflate or deflate the inflatable cells to the target pressure.

It will be appreciated that the support device, the sensor system and the steps described above are exemplary. For example, the sensor pad may comprise different types of sensing element. For example, the sensor pad may comprise different numbers and arrangements of sensing elements. For example, quantities other than frequency, such as voltage, that vary with the load applied to the sensing elements may be measured from the outputs of the sensing elements. For example, the load measurements may be combined in other ways to determine the 'sensed surface'. For example, the electric circuitry may not compare the count of the previous number of consecutive determinations to a threshold directly, but rather may determine the period of time associated with the count and compare the period of time to a threshold time value. For example, the electric circuitry may be configured to perform some of the steps described above. For example, the electric circuitry may be configured to perform additional steps.

## Claims

1. A support device for supporting the body of a person, the support device comprising:
an inflatable layer (20) formed of a plurality of inflatable cells (22) communicating with inflation and deflation means;
a sensor pad (102) arranged below the inflatable layer (20), the sensor pad (102) comprising at least three sensing elements (104) configured to sense the load applied to each sensing element (104), each sensing element (104) being a capacitive sensing element, the three sensing elements being separated from one another in a direction along the width of the support device, and the three sensing elements comprising:
a left sensing element arranged below a left hand portion of the inflatable layer,
a middle sensing element arranged below a central portion of the inflatable layer, and
a right sensing element arranged below a right hand portion of the inflatable layer; and
electric circuitry (108) connected to each sensing element (104) of the sensor pad (102), the electric circuitry (108) being configured to:
control the inflation and deflation means for filling or emptying, respectively, the inflatable cells (22) of the inflatable layer (20) in such a way that the internal air pressure of the inflatable cells (22) is a function of the load of the body of a person resting on the support device as sensed by each sensing element (104);
determine the position on the support device of a person resting on the support device based on the load sensed by each sensing element (104);
combine the load sensed by each sensing element and determine the total load of a person resting on the support device based on the combined load measurements;
compare the load sensed by the middle sensing element to the determined total load of a person resting on the support device; and
determine that the person resting on the support device is in an exit position on the support device if the load sensed by the middle sensing element is less than a predetermined fraction of the determined total load.

2. The support device according to claim 1, wherein the electric circuitry (108) is configured to determine the position on the support device of a person resting on the support device in the direction along the width of the support device, based on the load sensed by each sensing element (104).

3. The support device according to claims 1 or 2, wherein the electric circuitry (108) is configured to measure the load sensed by each sensing element (104) and compare the load sensed by each sensing element (104) to the loads sensed by the other sensing elements (104) to determine the position on the support device of a person resting on the support device.

4. The support device according to claims 1 or 2, wherein the electric circuitry (108) is further configured to determine movement of a person resting on the support device based on variations in the load sensed by each sensing element (104).

5. The support device according to claims 1 or 2, wherein the electric circuitry (108) is further configured to combine the loads sensed by the sensing elements (104) and determine movement of a person resting on the support device based on variations in the combined loads.

6. The support device according to any preceding claim, wherein each capacitive sensor comprises a flat condenser (200) comprising:
two layers of conductive material (201a, 201b); and
at least one layer of a compressible dielectric insulating material (203) arranged between the two layers of conductive material (201a, 201b).

7. The support device according to any preceding claim, wherein the support device further comprises a mattress comprising the inflatable layer (20) of inflatable cells (22).

8. The support device according to claim 7, wherein the sensor pad (102) is arranged within the mattress.

9. The support device according to claim 7, wherein the sensor pad (102) is integral with or attached to a lower surface of the mattress.

## Patentansprüche

1. Eine Trägervorrichtung zum Lagern des Körpers einer Person, wobei die Trägervorrichtung umfasst: eine aufblasbare Schicht (20), die aus einer Vielzahl von aufblasbaren Zellen (22) gebildet ist, die mit Mitteln zum Aufblasen und Luftablassen in Verbindung stehen;
eine Sensoreinlage (102), die unter der aufblasbaren Schicht (20) angeordnet ist, wobei die Sensoreinlage (102) mindestens drei Sensorelemente (104) umfasst, die so konfiguriert sind, dass sie die auf jedes Sensorelement (104) ausgeübte Belastung erfassen, wobei jedes Sensorelement (104) ein kapazitives Sensorelement ist, die drei Sensorelemente in einer Richtung entlang der Breite der Trägervorrichtung voneinander getrennt sind, und die drei Sensorelemente umfassen:
ein linkes Sensorelement, das unter einem linken Abschnitt der aufblasbaren Schicht angeordnet ist,
ein mittleres Sensorelement, das unter einem zentralen Abschnitt der aufblasbaren Schicht angeordnet ist, und
ein rechtes Sensorelement, das unter einem rechten Abschnitt der aufblasbaren Schicht angeordnet ist,
eine elektrische Schaltung (108), die mit jedem Sensorelement (104) der Sensoreinlage (102) verbunden ist, wobei die elektrische Schaltung (108) konfiguriert ist, um:
die Aufblas- und Luftablassvorrichtung zum Füllen bzw. Entleeren der aufblasbaren Zellen (22) der aufblasbaren Schicht (20) so zu steuern, dass der innere Luftdruck der aufblasbaren Zellen (22) eine Funktion der Last des Körpers einer auf der Trägervorrichtung ruhenden Person ist, wie sie von jedem Sensorelement (104) erfasst wird;
die Position einer auf der Trägervorrichtung ruhenden Person aufgrund der von jedem Sensorelement (104) erfassten Last zu bestimmen; die von jedem Sensorelement erfasste Last zusammenzufassen und die Gesamtlast einer auf der Trägervorrichtung ruhenden Person aufgrund der zusammengefassten Lastmessungen zu bestimmen;
die von dem mittleren Sensorelement erfasste Last mit der ermittelten Gesamtlast einer auf der Trägervorrichtung ruhenden Person zu vergleichen; und
zu bestimmen, dass sich die auf der Trägervorrichtung ruhende Person in einer Ausstiegsposition auf der Trägervorrichtung befindet, wenn die von dem mittleren Sensorelement erfasste Last geringer ist als ein vorbestimmter Bruchteil der ermittelten Gesamtlast.

2. Die Trägervorrichtung nach Anspruch 1, wobei die elektrische Schaltung (108) konfiguriert ist, um die Position einer auf der Trägervorrichtung ruhenden Person auf der Trägervorrichtung in der Richtung entlang der Breite der Trägervorrichtung aufgrund der von jedem Sensorelement (104) erfassten Last zu bestimmen.

3. Die Trägervorrichtung nach Anspruch 1 oder 2, wobei die elektrische Schaltung (108) konfiguriert ist, um die von jedem Sensorelement (104) erfasste Last zu messen und die von jedem Sensorelement (104) erfasste Last mit den von den anderen Sensorelementen (104) erfassten Lasten zu vergleichen, um die Position einer auf der Trägervorrichtung ruhenden Person zu bestimmen.

4. Die Trägervorrichtung nach Anspruch 1 oder 2, wobei die elektrische Schaltung (108) ferner so konfiguriert ist, dass sie die Bewegung einer auf der Trägervorrichtung ruhenden Person aufgrund von Variationen der von jedem Sensorelement (104) erfassten Last bestimmt.

5. Die Trägervorrichtung nach Anspruch 1 oder 2, wobei die elektrische Schaltung (108) ferner so konfiguriert ist, dass sie die von den Sensorelementen (104) erfassten Lasten zusammenfasst und die Bewegung einer auf der Trägervorrichtung ruhenden Person auf der Grundlage von Variationen der kombinierten Lasten bestimmt.

6. Die Trägervorrichtung nach einem der vorhergehenden Ansprüche, wobei jeder kapazitive Sensor einen Flachkondensator (200) umfasst, der Folgendes umfasst:
zwei Schichten aus leitfähigem Material (201a, 201b); und
mindestens eine Schicht aus einem komprimierbaren dielektrischen Isoliermaterial (203), die zwischen den beiden Schichten aus leitfähigem Material (201a, 201b) angeordnet ist.

7. Die Trägervorrichtung nach einem der vorhergehenden Ansprüche, wobei die Trägervorrichtung weiterhin eine Matratze umfasst, die die aufblasbare Schicht (20) aus aufblasbaren Zellen (22) umfasst.

8. Die Trägervorrichtung nach Anspruch 7, wobei die Sensoreinlage (102) innerhalb der Matratze angeordnet ist.

9. Die Trägervorrichtung nach Anspruch 7, wobei die Sensoreinlage (102) in eine untere Fläche der Matratze integriert oder an dieser befestigt ist.

## Revendications

1. Dispositif de support pour supporter le corps d'une personne, le dispositif de support comprenant : une couche gonflable (20) constituée d'une pluralité de cellules gonflables (22) communiquant avec des moyens de gonflage et de dégonflage ;
une plaquette de capteur (102) disposée sous la couche gonflable (20), la plaquette de capteur (102) comprenant au moins trois éléments de détection (104) configurés pour détecter la charge appliquée à chaque élément de détection (104), chaque élément de détection (104) étant un élément de détection capacitif, les trois éléments de détection étant séparés les uns des autres dans un sens le long de la largeur du dispositif de support, et les trois éléments de détection comprenant :
un élément de détection gauche disposé sous une partie gauche de la couche gonflable,
un élément de détection central disposé sous une partie centrale de la couche gonflable, et
un élément de détection droit disposé sous une partie droite de la couche gonflable ; et
un circuit électrique (108) connecté à chaque élément de détection (104) de la plaquette du capteur (102), le circuit électrique (108) étant configuré de manière à :
commander les moyens de gonflage et de dégonflage pour remplir ou vider, respectivement, les cellules gonflables (22) de la couche gonflable (20) de manière à ce que la pression d'air interne des cellules gonflables (22) représente une fonction de la charge du corps d'une personne reposant sur le dispositif de support telle que détectée par chaque élément de détection (104) ;
déterminer la position sur le dispositif de support d'une personne reposant sur le dispositif de support sur la base de la charge détectée par chaque élément de détection (104) ; combiner la charge détectée par chaque élément de détection et déterminer la charge totale d'une personne reposant sur le dispositif de support sur la base des mesures de charge combinées ;
comparer la charge détectée par l'élément de détection central à la charge totale déterminée d'une personne reposant sur le dispositif de support ; et déterminer que la personne reposant sur le dispositif de support est en position de sortie sur le dispositif de support si la charge détectée par l'élément de détection central est inférieure à une fraction prédéterminée de la charge totale déterminée.

2. Dispositif de support selon la revendication 1, dans lequel le circuit électrique (108) est configuré pour déterminer la position sur le dispositif de support d'une personne reposant sur le dispositif de support dans le sens le long de la largeur du dispositif de support, en fonction de la charge détectée par chaque élément de détection (104).

3. Dispositif de support selon les revendications 1 ou 2, dans lequel le circuit électrique (108) est configuré pour mesurer la charge détectée par chaque élément de détection (104) et comparer la charge détectée par chaque élément de détection (104) aux charges détectées par les autres éléments de détection (104) afin de déterminer la position sur le dispositif de support d'une personne reposant sur le dispositif de support.

4. Dispositif de support selon les revendications 1 ou 2, dans lequel le circuit électrique (108) est configuré pour déterminer le mouvement d'une personne reposant sur le dispositif de support sur la base des variations de la charge détectée par chaque élément de détection (104).

5. Dispositif de support selon les revendications 1 ou 2, dans lequel le circuit électrique (108) est en outre configuré pour combiner les charges détectées par les éléments de détection (104) et déterminer le mouvement d'une personne reposant sur le dispositif de support sur la base des variations des charges combinées.

6. Dispositif de support selon l'une quelconque des revendications précédentes, dans lequel chaque capteur capacitif comprend un condensateur plat (200) comprenant :
deux couches de matériau conducteur (201a, 201b) ; et
au moins une couche de matériau isolant diélectrique compressible (203) disposée entre les deux couches de matériau conducteur (201a, 201b).

7. Dispositif de support selon l'une quelconque des revendications précédentes, dans lequel le dispositif de support comprend en outre un matelas comprenant la couche gonflable (20) de cellules gonflables (22).

8. Dispositif de support selon la revendication 7, dans lequel la plaquette de capteur (102) est disposée à l'intérieur du matelas.

9. Dispositif de support selon la revendication 7, dans lequel la plaquette de capteur (102) est intégrée ou fixée à une surface inférieure du matelas.
